# EUROPEAN PATENT SPECIFICATION

(11) **EP 2 938 630 B1**
(45) Date of publication and mention of the grant of the patent: **02.08.2017**
(21) Application number: 13814133.8
(22) Date of filing: 20.12.2013
(51) Int. Cl.: C07K 14/64, C07K 19/00

(54) **FUSION POLYPEPTIDES AND USES THEREOF**
FUSIONSPOLYPEPTIDE UND VERWENDUNGEN DAVON
POLYPEPTIDES DE FUSION ET LEURS UTILISATIONS

(30) Priority: 27.12.2012 EP 12199488
(43) Date of publication of application: 04.11.2015
(73) Proprietor: Bayer Pharma Aktiengesellschaft, 13353 Berlin (DE)
(72) Inventor: WILMEN, Andreas, 50676 Köln (DE); LEINEWEBER, Kirsten, 42555 Velbert-Langenberg (DE); SCHEERER, Nina Alexandra, 44575 Castrop-Rauxel (DE); JÖRISSEN, Hannah, 42579 Heiligenhaus (DE)
(74) Representative: BIP Patents
(86) International application number: PCT/EP2013/077629
(87) International publication number: WO 2014/102179

(56) References cited:
- WO-A1-2013/004607
- WO-A1-2013/007563
- US-A1- 2011 243 942
- US-A1- 2012 046 229

## Description

The present invention provides Relaxin fusion polypeptides with extended half-life. Thereby, the half-life extending parts are either the Fc moiety of the human lgG2 or of the human IgG4. Furthermore, the invention provides nucleic acid sequences encoding the foregoing fusion polypeptides, vectors containing the same, pharmaceutical compositions and medical use of such fusion polypeptides.

### Background of the invention

Relaxin 2 (H2 relaxin, RLN2) as a member of the insulin superfamily is a 2-chain peptide exhibiting, on the genetic level, the typical B - C - A chain prohormone structure, arranged from N- to C-terminus. Other members of this superfamily, encoded by 7 genes in human, are the relaxin genes RLN 1, RLN3, and the insulin like peptide genes INSL3, INSL4, INSL5, and INSL6. The overall sequence homology between members of this family is low; nevertheless, phylogenetic analysis indicates that these genes have evolved from the RLN3 ancestral gene (Hsu, S. Y. (2003); Wilkinson, T. N. et al. (2005)). The mature protein has a molecular weight of approximately 6000 Da and is the product of an enzymatic cleavage of the prohormone catalyzed by the Prohormone-Convertase 1 (PC1) and 2 (PC2) (Hudson P. et al. (1983)). The resulting A- and B-chains are joined by two intermolecular cysteine bridges; the A-chain exhibits an additional intramolecular disulfide bond. Relaxin initiates pleiotropic effects through multiple pathways on a variety of cell types. It confers its activity by binding to the class I (rhodopsin like) G-protein-coupled receptor termed LGR7 (leucine-rich G protein-coupled receptor 7) also named RXFP1 (relaxin family peptide 1 receptor), and with significantly lower affinity to LRG8/RXFP2 (relaxin family peptide 2 receptor) (Kong RC et al. (2010) Mol Cell Endocrinol. 320:1-15). Within the Relaxin molecule, an amino acid motif in the B chain (Arg-X-X-X-Arg-X-X-Ile/Val-X) (Schwabe and Büllesbach (2007) Adv Exp Med Biol. 612:14-25 and Büllesbach and Schwabe J Biol Chem. 2000 Nov 10;275(45):35276-80) is conserved in all of the Relaxin peptides and is crucial for the interaction of these peptides with the corresponding receptor. Binding of Relaxin to LGR7/RXFP1 leads to activation of adenylate cyclase and to an increase of the second messenger molecule cAMP. Via this mechanism, Relaxin 2 for example mediates the release of atrial natriuretic peptide in rat hearts (Toth, M. et al. (1996)). A positive inotropic effect of Relaxin 2 on rat atrial myocytes has also been shown (Piedras-Renteria, E. S. et al. (1997)). Other signal transduction molecules which are activated by the Relaxin/LGR7 complex are the phosphoinositide-3 kinase, tyrosine kinases, and phosphodiesterases (Bartsch, O. et al. (2001), Bartsch, O. et al. (2004)). Additional signal transduction pathways activated by this system include the nitric oxide (NO) pathway leading to increased levels of cyclic GMP in rat and guineapig hearts (Bani-Sacchi, T. et al. (1995)).

Relaxin acts as a pleiotropic hormone (Dschietzig T. et al. (2006)) possessing biological activity on organs such as lung, kidney, brain, and heart. A strong antifibrotic and vasodilator activity of Relaxin is most notably responsible for the positive effects obtained with this peptide in various animal disease models as well as in clinical studies (McGuane J.T. et al. (2005)). RLN2 has multiple beneficial actions in the cardiovascular system under pathological conditions. It maintains tissue homeostasis and protects the injured myocardium during various pathophysiological processes. It exhibits prominent vasodilatory effects, e.g. affecting flow and vasodilation in rodent coronary arteries (Nistri, S. et al. (2003)) and in the vascular beds of other organs. In spontaneously hypertensive rats RLN2 lowered blood pressure, an effect mediated by increased NO production.

A cardioprotective activity of Relaxin 2 has been evaluated in different animal models such as guinea pig, rat and pig (Perna A.M. et al. (2005), Bani, D. et al. (1998)). RLN2 ameliorates myocardial injury, inflammatory cell infiltration and subsequent fibrosis, thereby alleviating severe ventricular dysfunction (Zhang J. et al. (2005)). Relaxin 2 exhibits strong antifibrotic activity. In injured tissues, fibroblast activation and proliferation causes increased collagen production and interstitial fibrosis. Fibrosis in the heart is increased by biomechanical overload, and influences ventricular dysfunction, remodeling, and arrhythmogenesis. In animal models, continuous infusion of Relaxin 2 inhibits or even reverses cardiac dysfunction caused by cardiomyopathy, hypertension, isoprenaline-induced cardiac toxicity, diabetic cardiomyopathy and myocardial infarction. This inhibition of fibrogenesis or reversal of established fibrosis can reduce ventricular stiffening and improve diastolic function. Notably, although Relaxin 2 reduces aberrant collagen accumulation, it does not affect basal collagen content in healthy tissues, highlighting its safety for therapeutic use.

Relaxin 2 has been tested in several clinical studies as a pleiotropic vasodilator for the treatment of patients with acute heart failure with very promising outcome. In these studies, Relaxin 2 was associated with favorable relief of dyspnoea and other clinical outcomes (Teerlink J.R. et al. (2009), Metra M. et al. (2010))
Due to the limited in-vivo half life of Relaxin, treatment of patients has to be repeated every 14 to 21 days, whereby compound administration has to be performed as a continuous infusion for at least 48 hours.
Furthermore, Relaxin 2 may also be useful in the treatment of diseases such as pancreatitis, inflammation-related diseases like rheumatoid arthritis, and cancer (Cosen-Binker L.I. et al. (2006) Santora K. Et al. (2007)) or scleroderma, pulmonary, renal, and hepatic fibrosis (Bennett RG. (2009)). Relaxin 2 reduces xenograft tumour growth of human MDA-MB-231 breast cancer cells (Radestock Y, Hoang-Vu C, Hombach-Klonisch S. (2008) Breast Cancer Res. 10:R71).

The synthesis of Relaxin 2 by chemical methods is difficult. Due to the low solubility of the B-chain and the requirement for the laborious, specific introduction of cysteine bridges between A and B-chains, yields of active peptide obtained by these methods are extremely low (Barlos K.K. et al. (2010)). Alternatively, recombinant expression of Relaxin 2 can be performed. To allow efficient cleavage of the prepro-peptide during post-translational modifications and the secretion of mature and biological active peptides, expression host cells are routinely co-transfected with expression constructs encoding the Prohormone-Convertase 1 and/or 2 (Park J.I. et al. (2008)). Nevertheless, the endoproteolytic processing efficiency of prepro-peptides in heterologous cells often limits the production of bioactive molecules significantly (Shaw J.A. et al. (2002)). The generation of a single chain Relaxin in which an uncleavable polypeptide connects the C-terminal end of the A-chain with the N-terminal end of B-chain results in a single chain Relaxin 2 molecule that exhibits full biological activity.

The half-life of intravenously administrated Relaxin 2 in humans is less than 10 minutes (Dschietzig T. et al. (2009)). As a consequence, in clinical trials Relaxin 2 has to be administered continuously over 48h. Therefore, the improvement of the biological half life of Relaxin could be of great advantage.
Improving biological half life can either be performed by chemical modification such as PEGylation or HESylation of the polypeptide of interest, introduction of additional, non-natural N-glycosylation sites or by genetically fusing this polypeptide with other molecules such as the immunoglobulin Fc fragment of antibodies, transferrin, albumin, binding modules that bind in-vivo to other molecules mediating longer half-life, or other proteins, respectively.
US20120046229 discloses in the sequence listing two non-functional single-chain polypeptides in which the A-chain and the B-chain of Relaxin 2 are fused to each other.
US20110243942 discloses fusion proteins in which Relaxins are fused to the N-terminus of IgG Fc-regions. The A- and B-chains of Relaxin are fused by a cleavable C-chain.
This invention provides single-chain Relaxin variants fused to the Fc part of the human Fc moiety of an IgG2 or IgG4 antibody with improved half-life.

### Summary of the invention

The invention concerns fusion polypeptides, hereafter also referred to a single chain Relaxin (scRelaxin) fused to the FC moieties of human IgG2 and human IgG4, respectively. These fusion proteins exhibit the biological activity of the non-modified, natural occurring Relaxin 2, but due to the fusion to one of the above mentioned IgG moieties these fusion proteins exhibit a prolonged serum half-life.

Using the Fc moieties of either human IgG2 or human IgG4 provide an advantage of having reduced antibody-dependent cell-mediated mytotoxicity (ADCC) or complement-dependent cytotoxicity (CDC).

### Brief description of the drawing

Figure 1 Activity of non-purified lgG2Fc-scRelaxin and IgG4Fc-scRelaxin in a functional assay using the CHO-CRELGR7 cell line. As control, hRelaxin 2 (R&D Systems, catalogue number 6586-RN-025) was used. Data are expressed as Relative Light Units, representing the activity of Fc-single chain Relaxin variants and Relaxin 2 induced luciferase expression. Symbols represent means, error bars represent S.E.M.
Figure 2 shows the polypeptide sequence of IgG2Fc-scRelaxin (SEQ ID NO:1). The stretcher sequence is in italic, the scRelaxin polypeptide sequence is underlined.
Figure 3 shows the polypeptide sequence of IgG4Fc-scRelaxin (SEQ ID NO:2).
Figure 4 shows the polynucleotide sequence of IgG2Fc-scRelaxin (SEQ ID NO:3).
Figure 5 shows the polynucleotide sequence of IgG4Fc-scRelaxin (SEQ ID NO:4).
Figure 6 shows a schematic representation of the genetic organization of domains of wildtype Relaxin and single chain Relaxin as well as their corresponding polypeptides.
Figure 7 shows a schematic representation of the domain organization of a single chain Relaxin fused to a Fc domain of IgG2 or IgG4, respectively.

### Detailed description of the invention

### Definitions:

The term "amino acid residue" is intended to indicate an amino acid residue contained in the group consisting of alanine (Ala or A), cysteine (Cys or C), aspartic acid (Asp or D), glutamic acid (Glu or E), phenylalanine (Phe or F), glycine (Gly or G), histidine (His or H), isoleucine (Ile or I), lysine (Lys or K), leucine (Leu or L), methionine (Met or M), asparagine (Asn or N), proline (Pro or P), glutamine (Gln or Q), arginine (Arg or R), serine (Ser or S), threonine (Thr or T), valine (Val or V), tryptophan (Trp or W), and tyrosine (Tyr or Y) residues.

The term "activity of Relaxin" or "Relaxin Acitvity" is defined by the ability of Relaxin or variants thereof to the activation of the stimulatory G-protein Gs, thus the subsequent generation of the second messenger cyclic AMP, and/or the stimulation of PI3-kinase. Relaxin or variants thereof bind to LGR7 leading to the intracellular activation of the stimulatory G-protein Gs, resulting in the subsequent generation of the second messenger cyclic AMP (cAMP). However, cAMP generation is a timedependent biphasic response. After an initial short Gs-adenylate cyclase-mediated cAMP response the receptor signal is switching to an inhibitory G protein activation and by this to PI3-kinase-mediated response. (Halls M.L., Bathgate R.A., Summers, R.J. (2005) Signal Switching after Stimulation of LGR7 Receptors by Human Relaxin 2. Ann. N.Y. Acad. Sci. 1041:288-291).

The term "half-life extending moiety" refers to a pharmaceutically acceptable moiety, domain, or "vehicle" covalently linked ("conjugated") to the Relaxin fusion polypeptide directly or via a linker, that prevents or mitigates in vivo proteolytic degradation or other activity-diminishing chemical modification of the Relaxin fusion polypeptide , increases half-life or other pharmacokinetic properties such as but not limited to increasing the rate of absorption, reducing clearance, reduces toxicity, improves solubility, increases biological activity and/or target selectivity of the Relaxin fusion polypeptide, increases manufacturability, and/or reduces immunogenicity of the Relaxin fusion polypeptide, compared to an unconjugated form of the Relaxin fusion polypeptide. The term "halflife extending moiety" includes proteinaceous half-life extending moieties, such as serum albumin, transferrin or Fc domain.

"Polypeptide", peptide" and "protein" are used interchangeably herein and include a molecular chain of two or more amino acids linked through peptide bonds. The terms do not refer to a specific length of the product. The terms include post-translational modifications of the polypeptide, for example, glycosylations, acetylations, phosphorylations and the like. In addition, protein fragments, analogs, mutated or variant proteins, fusion proteins and the like are included within the meaning of polypeptide. The terms also include molecules in which one or more amino acid analogs or non-canonical or unnatural amino acids are included as can be synthesized or expressed recombinantly using known protein engineering techniques. In addition, inventive fusion proteins can be derivatized as described herein by well-known organic chemistry techniques.

The term "functional variant" refers to a variant polypeptide which at least retains some of its natural biological activity. In case of the Relaxin 2 variants according to the invention, a functional variant is a variant which shows at least some of its natural activity, such as the activation of the relaxin receptor LGR7. The activation of the relaxin receptor LGR7 can be determined by a method disclosed in experimental methods.

The terms "fragment," "variant," "derivative," and "analog" when referring to polypeptides of the present invention include any polypeptides that retain at least some of the receptor binding properties of the corresponding wild-type Relaxin polypeptide. Fragments of polypeptides of the present invention include proteolytic fragments, as well as deletion fragments, and also polypeptides with altered amino acid sequences due to amino acid substitutions, deletions, or insertions. Variants may occur naturally or be non-naturally occurring. Non-naturally occurring variants may be produced using art-known mutagenesis techniques. Variant polypeptides may comprise conservative or non-conservative amino acid substitutions, deletions, or additions. Variant polypeptides may also be referred to herein as "polypeptide analogs." As used herein a "derivative" of a polypeptide refers to a subject polypeptide having one or more residues chemically derivatized by reaction of a functional side group. Also included as "derivatives" are those peptides that contain one or more naturally occurring amino acid derivatives of the twenty standard amino acids. For example, 4-hydroxyproline may be substituted for proline; 5-hydroxylysine may be substituted for lysine; 3-methylhistidine may be substituted for histidine; homoserine may be substituted for serine; and ornithine may be substituted for lysine.

The term "fusion protein" indicates that the protein includes polypeptide components derived from more than one parental protein or polypeptide and/or that the fusion protein includes protein domains derived from one or more parental protein or polypeptide which are not arrayed in their wild type orientation. Typically, a fusion protein is expressed from a fusion gene in which a nucleotide sequence encoding a polypeptide sequence from one protein is appended in frame with, and optionally separated by a linker or stretcher from, a nucleotide sequence encoding a polypeptide sequence from a different protein. The fusion gene can then be expressed by a recombinant host cell as a single protein.

The term "nucleotide sequence" or "polynucleotide "is intended to indicate a consecutive stretch of two or more nucleotide molecules. The nucleotide sequence may be of genomic, cDNA, RNA, semisynthetic, synthetic origin, or any combinations thereof.

The term "EC50" (half maximal effective concentration) refers to the effective concentration of a therapeutic compound which induces a response halfway between the baseline and maximum after some specified exposure time.

The term "immunogenicity" as used in connection with a given substance is intended to indicate the ability of the substance to induce a response from the immune system. The immune response may be a cell or antibody mediated response (see, e.g., Roitt: Essential Immunology (8th Edition, Black-well) for further definition of immunogenicity). Normally, reduced antibody reactivity will be an indication of reduced immunogenicity. The reduced immunogenicity may be determined by use of any suitable method known in the art, e.g. in vivo or in vitro.

The term "polymerase chain reaction" or "PCR" generally refers to a method for amplification of a desired nucleotide sequence in vitro, as described, for example, in US Pat. No. US 4,683,195 and US 4,683,195. In general, the PCR method involves repeated cycles of primer extension synthesis, using oligonucleotide primers capable of hybridizing preferentially to a template nucleic acid.

The term "vector" refers to a plasmid or other nucleotide sequences that are capable of replicating within a host cell or being integrated into the host cell genome, and as such, are useful for performing different functions in conjunction with compatible host cells (a vector-host system): to facilitate the cloning of the nucleotide sequence, i.e. to produce usable quantities of the sequence, to direct the expression of the gene product encoded by the sequence and to integrate the nucleotide sequence into the genome of the host cell. The vector will contain different components depending upon the function it is to perform.

"Cell", "host cell", "cell line" and "cell culture" are used interchangeably herein and all such terms should be understood to include progeny resulting from growth or culturing of a cell. The term "half-life" is used in its pharmacokinetic meaning as the time required for the drug concentration being reduced by one half compared to a given value *in vivo.* Half-life will usually be determined by concentration measurements in serum or plasma. Therefore, the alternative terms "serum half-life" or "plasma half-life" can be used to specify the originating matrix. For the determination of half-life not only the concentration but also the biological activity of the drug may be considered, which is then referred to as the "functional half-life" *in vivo,* meaning the time at which the activity of the polypeptide dropped by 50% of a given value. However, as determination of the drug concentration is often much easier that the determination of its biological activity in biological matrices, the serum half-life may be used as an indicator for its functional half-life *in vivo*.)

The functional half-life *in vivo* and the serum half-life may be determined by any suitable method known in the art and may for example generally involve the steps of suitably administering to a mammalian a suitable dose of the amino acid sequence or compound to be treated (preferably as an intravenous bolus); collecting blood samples or other samples from said mammalian at regular intervals and determining the level or concentration of the amino acid sequence or compound of the invention in said blood sample (preferably prepared as serum or plasma. The pharmacokinetic parameters such as for example Area-under-the curve (AUC), Cₘₐₓ, Clearance (CL) and particularly half-life can be calculated from concentration-time curves by compartmental or non-compartmental methods using validated pharmacokinetic software.
"Glycosylation" is a chemical modification wherein sugar moieties are added to the polypeptide at specific sites. Glycosylation of polypeptides is typically either N-linked or O-linked. N-linked refers to the attachment of a carbohydrate moiety to the side chain of an asparagine residue. The tripeptide sequences Asn-X-Ser and Asn-X-Thr ("N-X-S/T"), where X is any amino acid except proline, are the recognition sequences for enzymatic attachment of the carbohydrate moiety to the asparagine side chain. Thus, the presence of either of these tripeptide sequences (or motifs) in a polypeptide creates a potential N-linked glycosylation site. O-linked refers to the attachment of a carbohydrate moiety to the hydroxyl-group oxygen of serine and threonine.
An "isolated" fusion polypeptide is one that has been identified and separated from a component of the cell that expressed it. Contaminant components of the cell are materials that would interfere with diagnostic or therapeutic uses of the fusion polypeptide, and may include enzymes, hormones, and other proteinaceous or nonproteinaceous solutes. In preferred embodiments, the fusion polypeptide is purified (1) to greater than 95% by weight of fusion polypeptide as determined e.g. by the Lowry method, UV-Vis spectroscopy or by by SDS-Capillary Gel electrophoresis (for example on a Caliper LabChip GXII, GX 90 or Biorad Bioanalyzer device), and in further preferred embodiments more than 99% by weight, (2) to a degree sufficient to obtain at least 15 residues of N-terminal or internal amino acid sequence, or (3) to homogeneity by SDS-PAGE under reducing or non-reducing conditions using Coomassie blue or, preferably, silver stain. Ordinarily, however, isolated fusion polypeptides will be prepared by at least one purification step.

### Overview

### Half-life extension via proteinaceous half-life extending moieties:

**A** possibility for improving the half-life of a fusion scRelaxin 2 is a fusion with a proteinaceous half-life extending moiety, such as the immunoglobulin Fc fragment of antibodies. The scRelaxin polypeptides described above can be fused directly or via a peptide linker to the Fc portion of an immunoglobulin. "Immunoglobulins" are molecules containing polypeptide chains held together by disulfide bonds, typically having two light chains and two heavy chains. In each chain, one domain (V) has a variable amino acid sequence depending on the antibody specificity of the molecule. The other domains (C) have a rather constant sequence common to molecules of the same class.

As used herein, the "Fc" portion of an immunoglobulin has the meaning commonly given to the term in the field of immunology. Specifically, this term refers to an antibody fragment that is obtained by removing the two antigen binding regions (the Fab fragments) from the antibody. One way to remove the Fab fragments is to digest the immunoglobulin with papain protease. Thus, the Fc portion is formed from approximately equal sized fragments of the constant region from both heavy chains, which associate through non-covalent interactions and disulfide bonds. The Fc portion can include the hinge regions and extend through the CH2 and CH3 domains to the C-terminus of the antibody. Representative hinge regions for human and mouse immunoglobulins can be found in Antibody Engineering, A Practical Guide, Borrebaeck, C.A.K., ed., W.H. Freeman and Co., 1992.

There are five types of human immunoglobulin Fc regions with different effector and pharmacokinetic properties: IgG, IgA, IgM, IgD, and IgE. IgG is the most abundant immunoglobulin in serum. IgG also has the longest half-life in serum of any immunoglobulin (23 days). Unlike other immunoglobulins, IgG is efficiently recirculated following binding to an Fc receptor. There are four IgG subclasses G1, G2, G3, and G4, each of which has different effect or functions. These effector functions are generally mediated through interaction with the Fc receptor (FcγR) or by binding C1q and fixing complement. FcγRs are expressed on a panel of immune cells like natural killer cells (NK cells), monocytes, macrophages, or eosinophils , and formation of the Fc/FcγR complex recruits these cells to sites of bound antigen, typically resulting in signaling and subsequent immune responses such as release of inflammation mediators, B cell activation, endocytosis, phagocytosis, and cytotoxic attack. For example, binding to FcγR can lead to antibody dependent cell mediated cytotoxicity (ADCC), whereas binding to complement factors can lead to complement dependent cytotoxicity (CDC). In designing heterologous Fc fusion proteins wherein the Fc portion is being utilized solely for its ability to extend half-life, it can be an advantage to minimize the effector function, e.g. such as release of inflammatory mediators. All IgG subclasses are capable of binding to Fc receptors (CD16, CD32, CD64) with G1 and G3 being more effective than G2 and G4 (hIgG1 ≥ hIgG3 > hIgG2 ≥ IgG4).

Therefore, in certain instances (e.g. pathological condition of a patient) it is an advantage to use for a Relaxin fusion polypeptide the Fc domain of a human IgG2 or IgG4 antibody to reduce release of inflammation mediators, B cell activation, endocytosis, phagocytosis, and/or cytotoxic attack while still providing the benefit of a prolonged half-life of the Relaxin fusion polypeptide.

The Fc receptor binding region of IgG is formed by residues located in both the hinge and the carboxy terminal regions of the CH2 domain. Depending on the desired in vivo effect, the heterologous fusion proteins of the present invention may contain any of the isotypes described above or may contain mutated Fc regions wherein the complement and/or Fc receptor binding functions have been altered. Thus, the heterologous fusion proteins of the present invention may contain the entire Fc portion of an immunoglobulin, fragments of the Fc portion of an immunoglobulin, or analogs thereof fused to a scRelaxin compound. Regardless of the final structure of the fusion protein, the Fc or Fc-like region must serve to prolong the in vivo plasma half-life of the scRelaxin compound fused at the C-terminus or N-terminus. Preferably, the fused scRelaxin compound retains some biological activity. Biological activity can be determined by in vitro and in vivo methods known in the art. It is preferable that the Fc region used for the heterologous fusion proteins of the present invention be derived from an lgG2 or an lgG4 Fc region.

Generally, the Fc region used for the heterologous fusion proteins of the present invention can be derived from any species including but not limited to human, rat, mouse and pig. Preferably, the Fc region used for the present invention is derived from human or rat. However, most preferred are human Fc regions and fragments and variants thereof to reduce the risk of the fusion protein being immunogenic in humans. A "native sequence Fc region" comprises an amino acid sequence identical to the amino acid sequence of an Fc region found in nature. A "variant Fc region" comprises an amino acid sequence which differs from that of a native sequence Fc region by virtue of at least one amino acid modification. Preferably, the variant Fc region has at least one amino acid substitution compared to a native sequence Fc region or to the Fc region of a parent polypeptide, e.g., from about one to about ten amino acid substitutions, and preferably from about one to about five amino acid substitutions in a native sequence Fc region or in the Fc region of the parent polypeptide. The variant Fc region herein will preferably possess at least about 80% sequence identity with a native sequence Fc region and/or with an Fc region of a parent polypeptide, and most preferably at least about 90% sequence identity therewith, more preferably at least about 95% sequence identity therewith. Fc fusion polypeptides can form dimers. Preferred dimers are homodimers.

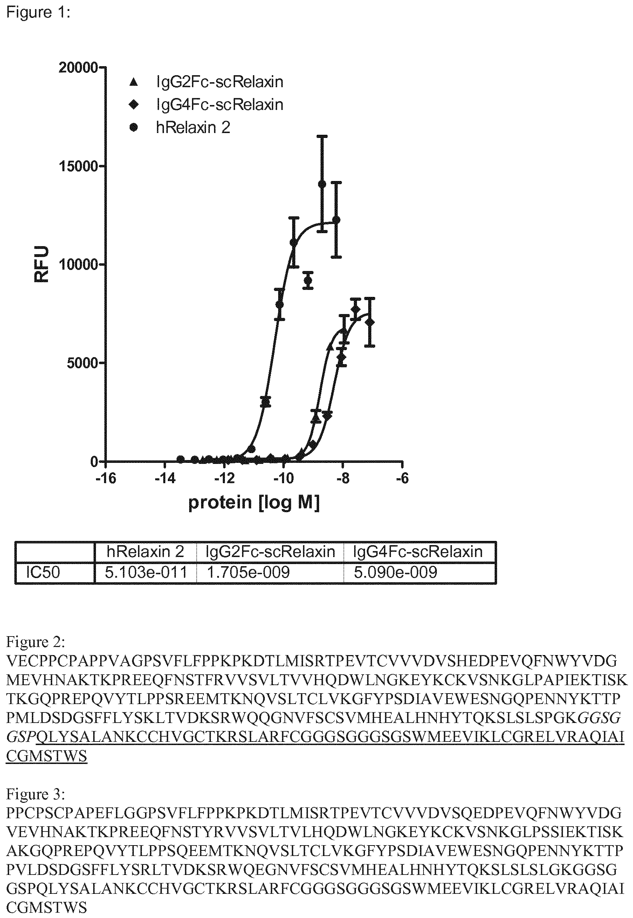

A preferred embodiment of the disclosure is a fusion polypeptide comprising a Fc domain of a human lgG2 or lgG4 antibody fused via its carboxy-terminus to the scRelaxin molecule (the scRelaxin molecule has the underlined amino acid sequence of Figure 2). A further preferred embodiment of the disclosure is a fusion polypeptide comprising a Fc domain of a human lgG2 or lgG4 antibody fused via a stretcher unit to the scRelaxin molecule.

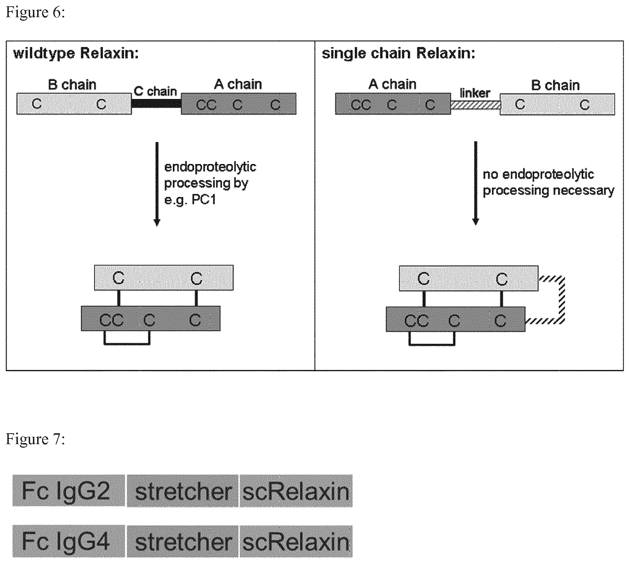

In a more preferred embodiment the fusion polypetide comprises in order from the amino-terminus to the carboxy-terminus (as depicted in Figure 7):
a) a human lgG2 or lgG4 Fc domain,
b) a stretcher unit, and
c) the scRelaxin

In a more preferred embodiment the fusionpolypetide comprises in order from the amino-terminus to the carboxy-terminus (as depicted in Figure 7):
a) a human IgG2 or IgG4 Fc domain,
b) a stretcher unit as depicted in Figure 2, and
c) the scRelaxin

A further preferred embodiment is a homodimer of a polypeptide as depicted above.

### Stretcher Units:

Such stretchers are known in the art and are 1 to about 100 amino acids in length, are 1 to about 50 amino acids in length, are 1 to about 25 amino acids in length, are 1 to about 15 amino acids in length, are 1 to 10 amino acids in length, are 4 to 25 amino acids in length, are 4 to 20 amino acids in length, are 4 to 15 amino acids in length, or are 4 to 10 amino acids in length.
The amino acid composition of stretcher sequences is variable, although a stretcher exhibiting a low immunogenicity score is preferred. In an embodiment of the invention a stretcher polypeptide connecting the scRelaxin 2 with a proteinaceous half-life extending moiety can be composed of any amino acid.
In a preferred embodiment the stretcher polypeptide comprises at least one Gly, Ser, Ile, Glu, Arg, Met, and/or Asp residue. In a more preferred embodiment the stretcher polypeptide comprises Gly and Ser residues. In a further preferred embodiment the stretcher peptide is a glycine-rich linker such as peptides comprising the sequence [GGGGS]n as disclosed in U.S. Patent No. 7,271,149. In other embodiments, a serine-rich strecher polypeptide is used, as described in U.S. Patent No. 5,525,491. A further preferred embodiment is a stretcher polypeptide which comprises Gly and Ser residues and has a ratio of Gly to Ser of at least 3 to 1.

In a preferred embodiment the proteinaceous half-life extending moiety is either the Fc moiety from a human lgG2 or lgG4 antibody.

In a further embodiment the aforementioned fusion polypeptides further comprising at least one half-life extending moiety have an extended half-life compared to the corresponding wild type Relaxin, wherein the half-life extension is at least 5, 10, 20, 50, 100 or 500-fold. Preferably, the half-life is determined as serum or plasma half-life, meaning detection of the fusion protein in serum or plasma, for example by using a commercially available quantification ELISA assay (e.g. R&D Systems, Human Relaxin-2 Quantikine ELISA kit, catalogue number DRL200) or other suitable assays for quantification. The half-life is preferably a human serum or plasma half-life. Preferably, the half-life is also determined as functional half-life in vivo, meaning the activity of fusion polypeptide in serum or plasma samples is determined. Assays to determine the activity of a scRelaxin of the invention are known in the art and are described herein.

### Cloning, vector systems, expression, hosts, and purification

The invention also provides for a vector which comprises an isolated nucleic acid molecule encoding a fusion polypeptide of the invention. This vector system is operatively linked to an expression sequence capable of directing its expression in a host cell.

A suitable host cell may be selected from the group consisting of bacterial cells (such as E. coli), yeast cells (such as Saccharomyces cerevisiae), fungal cells, plant cells, insect cells and animals cells. Animal cells include, but are not limited to, HEK293 cells, CHO cells, COS cells, BHK cells, HeLa cells and various primary mammalian cells. Derivatives of mammalian cells such as HEK293T cells are also applicable.

### DNA molecules of the invention

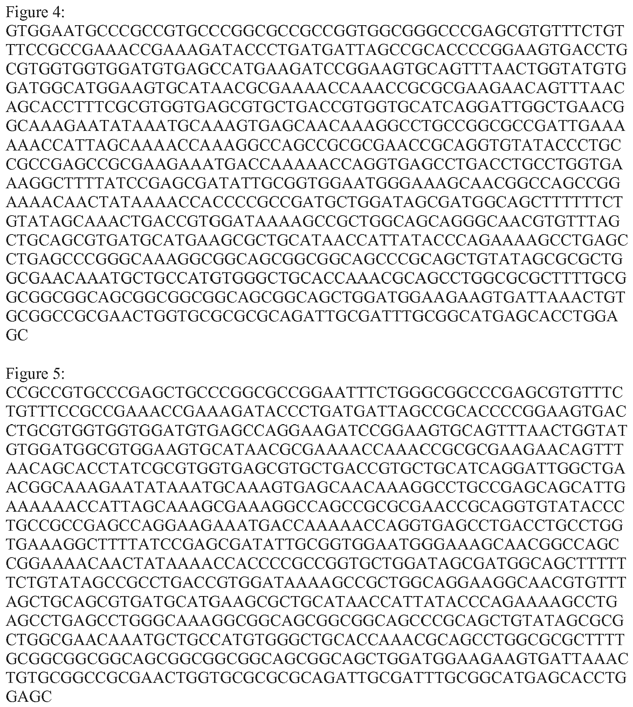

The present invention also relates to the DNA molecules that encode a fusion protein of the invention. Preferred sequences are shown in Figures 4 and 5.

DNA molecules of the disclosure are not limited to the sequences disclosed herein, but also include variants thereof. DNA variants within the invention may be described by reference to their physical properties in hybridization. The skilled worker will recognize that DNA can be used to identify its complement and, since DNA is double stranded, its equivalent or homolog, using nucleic acid hybridization techniques. It also will be recognized that hybridization can occur with less than 100% complementarity. However, given appropriate choice of conditions, hybridization techniques can be used to differentiate among DNA sequences based on their structural relatedness to a particular probe. For guidance regarding such conditions see, Sambrook et al., 1989 supra and Ausubel et al., 1995 (Ausubel, F. M., Brent, R., Kingston, R. E., Moore, D. D., Sedman, J. G., Smith, J. A., & Struhl, K. eds. (1995). Current Protocols in Molecular Biology. New York: John Wiley and Sons). Structural similarity between two polynucleotide sequences can be expressed as a function of "stringency" of the conditions under which the two sequences will hybridize with one another. As used herein, the term "stringency" refers to the extent that the conditions disfavor hybridization. Stringent conditions strongly disfavor hybridization, and only the most structurally related molecules will hybridize to one another under such conditions. Conversely, non-stringent conditions favor hybridization of molecules displaying a lesser degree of structural relatedness.

Hybridization stringency, therefore, directly correlates with the structural relationships of two nucleic acid sequences. The following relationships are useful in correlating hybridization and relatedness (where Tm is the melting temperature of a nucleic acid duplex):
a. Tm = 69.3 + 0.41(G+C)%
b. The Tm of a duplex DNA decreases by 1°C with every increase of 1% in the number of mismatched base pairs.
c. (Tm)µ2 - (Tm) µ1 = 18.5 log10µ2/µ1
where µ1 and µ2 are the ionic strengths of two solutions.

Hybridization stringency is a function of many factors, including overall DNA concentration, ionic strength, temperature, probe size and the presence of agents which disrupt hydrogen bonding. Factors promoting hybridization include high DNA concentrations, high ionic strengths, low temperatures, longer probe size and the absence of agents that disrupt hydrogen bonding. Hybridization typically is performed in two phases: the "binding" phase and the "washing" phase.

First, in the binding phase, the probe is bound to the target under conditions favoring hybridization. Stringency is usually controlled at this stage by altering the temperature. For high stringency, the temperature is usually between 65°C and 70°C, unless short (< 20 nt) oligonucleotide probes are used. A representative hybridization solution comprises 6X SSC, 0.5% SDS, 5X Denhardt's solution and 100 µg of nonspecific carrier DNA. See Ausubel et al., section 2.9, supplement 27 (1994). Of course, many different, yet functionally equivalent, buffer conditions are known. Where the degree of relatedness is lower, a lower temperature may be chosen. Low stringency binding temperatures are between about 25°C and 40°C. Medium stringency is between at least about 40°C to less t han about 65°C. High stringency is at least about 65°C.

Second, the excess probe is removed by washing. It is at this phase that more stringent conditions usually are applied. Hence, it is this "washing" stage that is most important in determining relatedness via hybridization. Washing solutions typically contain lower salt concentrations. One exemplary medium stringency solution contains 2X SSC and 0.1% SDS. A high stringency wash solution contains the equivalent (in ionic strength) of less than about 0.2X SSC, with a preferred stringent solution containing about 0.1X SSC. The temperatures associated with various stringencies are the same as discussed above for "binding." The washing solution also typically is replaced a number of times during washing. For example, typical high stringency washing conditions comprise washing twice for 30 minutes at 55°C. and three times for 15 minutes at 60°C.

An embodiment of the invention is an isolated nucleic acid sequence that encodes a fusion polypeptide of the invention.

Recombinant DNA constructs and expression The present invention further provides recombinant DNA constructs comprising one or more of the nucleotide sequences of the present invention. The recombinant constructs of the present invention are used in connection with a vector, such as a plasmid, phagemid, phage or viral vector, into which a DNA molecule encoding a fusion polypeptide of the invention is inserted.

A fusion polypeptide as provided herein can be prepared by recombinant expression of nucleic acid sequences encoding a fusion polypeptide in a host cell. To express a fusion polypeptide recombinantly, a host cell can be transfected with a recombinant expression vectors carrying DNA fragments encoding a fusion polypeptide such that the fusion polypeptide is expressed in the host cell. Standard recombinant DNA methodologies are used to prepare and/or obtain nucleic acids encoding a fusion polypeptide, incorporate these nucleic acids into recombinant expression vectors and introduce the vectors into host cells, such as those described in Sambrook, Fritsch and Maniatis (eds.), Molecular Cloning; A Laboratory Manual, Second Edition, Cold Spring Harbor, N.Y., (1989), Ausubel, F. M. et al. (eds.) Current Protocols in Molecular Biology, Greene Publishing Associates, (1989) and in U.S. Pat. No. 4,816,397 by Boss et al.

To express the fusion polypeptide standard recombinant DNA expression methods can be used (see, for example, Goeddel; Gene Expression Technology. Methods in Enzymology 185, Academic Press, San Diego, Calif. (1990)). For example, DNA encoding the desired polypeptide can be inserted into an expression vector which is then transfected into a suitable host cell. Suitable host cells are prokaryotic and eukaryotic cells. Examples for prokaryotic host cells are e.g. bacteria, examples for eukaryotic host cells are yeast, insect or mammalian cells. It is understood that the design of the expression vector, including the selection of regulatory sequences is affected by factors such as the choice of the host cell, the level of expression of protein desired and whether expression is constitutive or inducible.

### Bacterial Expression

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and, if desirable, to provide amplification within the host. Suitable prokaryotic hosts for transformation include E. coli, Bacillus subtilis, Salmonella typhimurium and various species within the genera Pseudomonas, Streptomyces, and Staphylococcus.

Bacterial vectors may be, for example, bacteriophage-, plasmid- or phagemid-based. These vectors can contain a selectable marker and bacterial origin of replication derived from commercially available plasmids typically containing elements of the well known cloning vector pBR322 (ATCC 37017). Following transformation of a suitable host strain and growth of the host strain to an appropriate cell density, the selected promoter is de-repressed/induced by appropriate means (e.g., temperature shift or chemical induction) and cells are cultured for an additional period. Cells are typically harvested by centrifugation, disrupted by physical or chemical means, and the resulting crude extract retained for further purification.

In bacterial systems, a number of expression vectors may be advantageously selected depending upon the use intended for the protein being expressed. For example, when a large quantity of such a protein is to be produced vectors which direct the expression of high levels of fusion polypeptide products that are readily purified may be desirable. Fusion polypeptide of the present invention include purified products, products of chemical synthetic procedures, and products produced by recombinant techniques from a prokaryotic host, including, for example, E. coli, Bacillus subtilis, Salmonella typhimurium and various species within the genera Pseudomonas, Streptomyces, and Staphylococcus, preferably, from E. coli cells.

### Eukaryotic Expression

Eukaryotic cells can be used to express the polypeptides of the invention. Systems for expression of proteins are known in the art. Such systems include e.g. include the eukaryotic cell, growth media, and corresponding expression vectors. Common eukaryotic cells for expression are e.g. a mammalian cell, a yeast cell, a plant cell, or an insect cell.

### Mammalian Expression and Purification

Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. For further description of viral regulatory elements, and sequences thereof, see e.g., U.S. 5,168,062 by Stinski, U.S. 4,510,245 by Bell et al. and U.S. 4,968,615 by Schaffner et al. The recombinant expression vectors can also include origins of replication and selectable markers (see e.g., U.S. 4,399,216, 4,634,665 and U.S. 5,179,017, by Axel et al.). Suitable selectable markers include genes that confer resistance to drugs such as G418, hygromycin or methotrexate, on a host cell into which the vector has been introduced. For example, the dihydrofolate reductase (DHFR) gene confers resistance to methotrexate and the neo gene confers resistance to G418. Transfection of the expression vector into a host cell can be carried out using standard techniques such as electroporation, calcium-phosphate precipitation, and DEAE-dextran, lipofection or polycation-mediated transfection.

Suitable mammalian host cells for expressing the fusion polypeptides provided herein include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, e.g., as described in R. J. Kaufman and P. A. Sharp (1982) Mol. Biol. 159:601-621, NSO myeloma cells, COS cells and SP2 cells. In some embodiments, the expression vector is designed such that the expressed protein is secreted into the culture medium in which the host cells are grown. Transient transfection/expression of antibodies can for example be achieved following the protocols by Durocher et al (2002) Nucl.Acids Res. Vol 30 e9. Stable transfection/expression of antibodies can for example be achieved following the protocols of the UCOE system (T. Benton et al. (2002) Cytotechnology 38: 43-46). The fusion polypeptide can be recovered from the culture medium using standard protein purification methods.

A fusion polypeptide of the invention can be recovered and purified from recombinant cell cultures by well-known methods including, but not limited to ammonium sulfate or ethanol precipitation, acid extraction, Protein A chromatography, Protein G chromatography, anion or cation exchange chromatography, phospho-cellulose chromatography, hydrophobic interaction chromatography, affinity chromatography, hydroxylapatite chromatography and lectin chromatography. High performance liquid chromatography ("HPLC") can also be employed for purification. See, e.g., Colligan, Current Protocols in Immunology, or Current Protocols in Protein Science, John Wiley & Sons, NY, N.Y., (1997-2001), e.g., Chapters 1, 4, 6, 8, 9, 10, each entirely incorporated herein by reference.

Fusion polypeptides of the invention include purified or isolated products, products of chemical synthetic procedures, and products produced by recombinant techniques from a eukaryotic host, including, for example, yeast (for example Pichia), higher plant, insect and mammalian cells, preferably from mammalian cells. Depending upon the host employed in a recombinant production procedure, the fusion polypeptide of the present invention can be glycosylated or can be non-glycosylated, with glycosylated preferred. Such methods are described in many standard laboratory manuals, such as Sambrook, supra, Sections 17.37-17.42; Ausubel, supra, Chapters 10, 12, 13, 16, 18 and 20.

A preferred homodimer of the invention is obtainable by transforming a host cell with a vector according comprising a polynucleotide of the invention, preferably a polynucleotide comprising SEQ ID NO 3 or 4, allowing expression of a fusion polypeptide of the invention, preferably a polypeptide comprising SEQ ID NO: 1 or 2, cultivating the host cells under conditions allowing expression of said fusion polypeptide and formation of the homodimer, and isolating the homodimer. Conditions allowing for the formation of a homodimer are conditions which allow the formation of interchain disulfide bonds between a fusion polypeptide of the invention. Such conditions are for example in Eukaryotic host cell. A preferred host cell for the process is a Eukaryotic host cell, more preferred is a mammalian host cell.

### Therapeutic Use

An embodiment of the invention is a pharmaceutical composition or a fusion polypeptide of the invention for use in the treatment of cardiovascular diseases, kidney diseases, pancreatitis, inflammation, cancer, scleroderma, pulmonary, renal, and hepatic fibrosis.

### Cardiovascular Diseases

Disorders of the cardiovascular system, or cardiovascular disorders, mean in the context of the present invention for example the following disorders: hypertension (high blood pressure), peripheral and cardiac vascular disorders, coronary heart disease, stable and unstable angina pectoris, myocardial insufficiency, persistent ischemic dysfunction ("hibernating myocardium"), temporary postischemic dysfunction ("stunned myocardium"), heart failure, disturbances of peripheral blood flow, acute coronary syndrome, heart failure and myocardial infarction.

In the context of the present invention, the term heart failure includes both acute and chronic manifestations of heart failure with reduced ejection fraction (HFrEF) and heart failure with preserved ejection fraction (HFpEF), as well as more specific or related types of disease, such as acute decompensated heart failure, right heart failure, left heart failure, global failure, ischemic cardiomyopathy (ICM), dilated cardiomyopathy (DCM), hypertrophic cardiomyopathy (HCM), congenital heart defects, heart valve defects, heart failure associated with heart valve defects, mitral stenosis, mitral insufficiency, aortic stenosis, aortic insufficiency, tricuspid stenosis, tricuspid insufficiency, pulmonary stenosis, pulmonary valve insufficiency, combined heart valve defects, myocardial inflammation (myocarditis), chronic myocarditis, acute myocarditis, viral myocarditis, diabetic heart failure, alcoholic cardiomyopathy, cardiac storage and metabolic disorders, and acute phases of worsening heart failure.

Preferred heart failure diseases are selected from the group of diseases comprising heart failure with reduced ejection fraction (HFrEF), heart failure with preserved ejection fraction (HFpEF), acute decompensated heart failure, right heart failure, left heart failure, global failure, ischemic cardiomyopathy (ICM), dilated cardiomyopathy (DCM), hypertrophic cardiomyopathy (HCM), and congenital heart defects.

The compounds according to the invention are further also suitable for reducing the area of myocardium affected by an infarction, the adverse cardiac remodeling after myocardial infarction and for the prophylaxis of secondary infarctions.

The compounds according to the invention are furthermore suitable for the prophylaxis and/or treatment of thromboembolic disorders, reperfusion damage following ischemia, micro- and macrovascular lesions (vasculitis), arterial and venous thromboses, edemas, ischemias such as myocardial infarction, stroke and transient ischemic attacks, for cardio protection in connection with coronary artery bypass operations (CABG), primary percutaneous transluminal coronary angioplasties (PTCAs), PTCAs after thrombolysis, rescue PTCA, heart transplants and open-heart operations, and for organ protection in connection with transplants, bypass operations, catheter examinations and other surgical procedures. Other areas of indication are, for example, the prevention and/or treatment of respiratory disorders, such as, for example, chronic obstructive pulmonary disease (chronic bronchitis, COPD), asthma, pulmonary emphysema, bronchiectases, cystic fibrosis (mucoviscidosis) and pulmonary hypertension, in particular pulmonary arterial hypertension.

### Kidney disease

The present invention relates to a fusion polypeptide of the invention for use as a medicament for the prophylaxis and/or treatment of kidney diseases, especially of acute and chronic kidney diseases and acute and chronic renal insufficiencies, as well as acute and chronic renal failure, including acute and chronic stages of renal failure with and without the requirement of dialysis, as well as the underlying or related kidney diseases such as renal hypoperfusion, dialysis induced hypotension, glomerulopathies, glomerular and tubular proteinuria, renal edema, hematuria, primary, secondary, as well as acute and chronic glomerulonephritis, membranous and membranoproliferative glomerulonephritis, Alport-Syndrome, glomerulosclerosis, interstistial tubular diseases, nephropathic diseases, such as primary and inborn kidney diseases, renal inflammation, immunological renal diseases like renal transplant rejection, immune complex induced renal diseases, as well as intoxication induced nephropathic diseases, diabetic and non-diabetic renal diseases, pyelonephritis, cystic kidneys, nephrosclerosis, hypertensive nephrosclerosis, nephrotic syndrome, that are characterized and diagnostically associated with an abnormal reduction in creatinine clearance and/or water excretion, abnormal increased blood concentrations of urea, nitrogen, potassium and/or creatinine, alteration in the activity of renal enzymes, such as glutamylsynthetase, urine osmolarity and urine volume, increased microalbuminuria, macroalbuminuria, glomerular and arteriolar lesions, tubular dilation, hyperphosphatemia and /or the requirement of dialysis.

In addition, a fusion polypeptide of the invention can be used as a medicament for the prophylaxis and/or treatment of renal carcinomas, after incomplete resection of the kidney, dehydration after overuse of diuretics, uncontrolled blood pressure increase with malignant hypertension, urinary tract obstruction and infection, amyloidosis, as well as systemic diseases associated with glomerular damage, such as Lupus erythematodes, and rheumatic immunological systemic diseases, as well as renal artery stenosis, renal artery thrombosis, renal vein thrombosis, analgetics induced nephropathy and renal tubular acidosis.

In addition, a fusion polypeptide of the invention can be used as a medicament for the prophylaxis and/or treatment of contrast medium induced and drug induced acute and chronic interstitial kidney diseases, metabolic syndrome and dyslipemia.

In addition, the present invention includes the use of a fusion polypeptide of the invention as a medicament for the prophylaxis and/or treatment of aftereffects associated with acute and/or chronic kidney diseases, such as pulmonary edema, heart failure, uremia, anemia, electrolyte disturbances (e.g. hyperkalemia, hyponatremia), as well as bony and carbohydrate metabolism.

### Lung Diseases

Furthermore, the fusion polypeptides according to the invention are also suitable for the treatment and/or prophylaxis of lung diseases especially of asthmatic disorders, pulmonary arterial hypertension (PAH) and other forms of pulmonary hypertension (PH) including left-heart disease, HIV, sickle cell anaemia, thromboembolisms (CTEPH), sarkoidosis, COPD or pulmonary fibrosis-associated pulmonary hypertension, chronic-obstructive pulmonary disease (COPD), acute respiratory distress syndrome (ARDS), acute lung injury (ALI), alpha-1-antitrypsin deficiency (AATD), pulmonary fibrosis, pulmonary emphysema (for example pulmonary emphysema induced by cigarette smoke) and cystic fibrosis (CF).

### Fibrotic Disorders

The fusion polypeptides according to the invention are furthermore suitable for the treatment and/or prophylaxis of fibrotic disorders of the internal organs such as, for example, the lung, the heart, the kidney, the bone marrow and in particular the liver, and also dermatological fibroses and fibrotic eye disorders. In the context of the present invention, the term fibrotic disorders includes in particular the following terms: hepatic fibrosis, cirrhosis of the liver, pulmonary fibrosis, myocardial fibrosis, nephropathy, glomerulonephritis, interstitial renal fibrosis, fibrotic damage resulting from diabetes, bone marrow fibrosis and similar fibrotic disorders, scleroderma, morphea, keloids, hypertrophic scarring (also following surgical procedures), naevi, diabetic retinopathy, proliferative vitreoretinopathy and disorders of the connective tissue (for example sarcoidosis).

### Cancer

Cancer is disease in which a group of cells display uncontrolled growth. Cancers are usually classified in carcinomas which is a cancer derived from epithelial cells (This group includes many of the most common cancers, including those of the breast, prostate, lung and colon.); sarcomas, which are derived from connective tissue, or mesenchymal cells; lymphoma and leukemia, derived from hematopoietic cells; germ cell tumor, which is derived from pluripotent; and blastomas, which is a cancer derived from immature "precursor" or embryonic tissue.

The present invention furthermore provides the use of a fusion polypeptide of the invention for preparing a medicament for the treatment and/or prevention of disorders, in particular the disorders mentioned above.

The present invention furthermore provides a method for the treatment and/or prevention of disorders, in particular the disorders mentioned above, using an effective amount of at least one fusion polypeptide of the invention.

The present invention furthermore provides a fusion polypeptide of the invention for use in a method for the treatment and/or prophylaxis of coronary heart disease, acute coronary syndrome, heart failure, and myocardial infarction.

### Pharmaceutical Compositions and Administration

The present invention also provides for pharmaceutical compositions comprising a single chain Relaxin fusion protein in a pharmacologically acceptable vehicle. The single chain Relaxin fusion protein may be administrated systemically or locally. Any appropriate mode of administration known in the art may be used including, but not limited to, intravenous, intraperitoneal, intraarterial, intranasal, by inhalation, oral, subcutaneous administration, by local injection or in form of a surgical implant. The present invention also relates to pharmaceutical compositions which may comprise inventive fusion polypeptides, alone or in combination with at least one other agent, such as stabilizing compound, which may be administered in any sterile, biocompatible pharmaceutical carrier, including, but not limited to, saline, buffered saline, dextrose, and water. Any of these molecules can be administered to a patient alone, or in combination with other agents, drugs or hormones, in pharmaceutical compositions where it is mixed with excipient(s) or pharmaceutically acceptable carriers. In one embodiment of the present invention, the pharmaceutically acceptable carrier is pharmaceutically inert.

### Therapeutically Effective Dose

Pharmaceutical compositions suitable for use in the present invention include compositions wherein the active ingredients are contained in an effective amount to achieve the intended purpose, e.g. heart failure. The determination of an effective dose is well within the capability of those skilled in the art.

For any compound, the therapeutically effective dose can be estimated initially either in in vitro assays, e.g. LGR7 receptor activation, ex vivo in isolated perfused rat hearts, or in animal models, usually mice, rabbits, dogs, or pigs. The animal model is also used to achieve a desirable concentration range and route of administration.

Such information can then be used to determine useful doses and routes for administration in humans.

A therapeutically effective dose refers to that amount of fusion polypeptide that ameliorates the symptoms or condition. Therapeutic efficacy and toxicity of such compounds can be determined by standard pharmaceutical procedures in vitro or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, ED50/LD50. Pharmaceutical compositions that exhibit large therapeutic indices are preferred. The data obtained from in vitro assays and animal studies are used in formulating a range of dosage for human use. The dosage of such compounds lies preferably within a range of circulating concentrations what include the ED50 with little or no toxicity. The dosage varies within this range depending upon the dosage form employed, sensitivity of the patient, and the route of administration.

Normal dosage amounts may vary from 0.1 to 100,000 milligrams total dose, depending upon the route of administration. Guidance as to particular dosages and methods of delivery is provided in the literature. See U.S. Pat. No. 4,657,760; 5,206,344; or 5,225,212. Those skilled in the art will employ different formulations for polynucleotides than for proteins or their inhibitors. Similarly, delivery of polynucleotides or polypeptides will be specific to particular cells, conditions, locations, etc.

The present invention is further described by the following examples. The examples are provided solely to illustrate the invention by reference to specific embodiments.

These exemplifications, while illustrating certain specific aspects of the invention, do not portray the limitations or circumscribe the scope of the disclosed invention. All examples were carried out using standard techniques, which are well known and routine to those of skill in the art, except where otherwise described in detail. Routine molecular biology techniques of the following examples can be carried out as described in standard laboratory manuals, such as Sambrook et al., Molecular Cloning: A Laboratory Manual, 2nd Ed.; Cold Spring Harbor Laboratory Press, Cold Spring Harbor, N.Y., 1989.

### Examples

### Experimental protocols

### Construction of Relaxin variants:

The cDNA sequences of the Relaxin variants were generated by chemical gene synthesis. The synthesized genes were subcloned into the mammalian expression vector pCEP4 (Invitrogen, catalogue number V044-50). As signal leader sequence for correct secretion of the resulting protein, either the leader sequence of the LDL receptor-related protein (LRP, amino acid composition MLTPPLLLLLPLLSALVAA) or of CD33 (amino acid composition MPLLLLLPLLWAGALA) were used. For subcloning of the synthesized constructs the restriction enzymes Hindlll and BamH1 were used according to manufactures' instruction.

### Expression of Relaxin variants:

For small scale expression (up to 2 milliliter culture volume) HEK293 (ATCC, catalogue number CRL-1573) cells were transiently transfected using Lipofectamine2000 Transfection Reagent (Invitrogen, catalogue number 11668-019) according to manufactures' Instructions. Cells were cultivated in D-Mem F12 (Gibco, #31330), 1% Penicillin-Streptomycin (Gibco, #15140) and 10% fetal calf serum (FCS, Gibco, #11058) in a humified incubator at 5% carbon dioxide at 37°C.

Three to five days following transfection, conditioned medium of the transfected cells were tested for activity using the stably transfected CHO-CRE-GR7 cell line. For large scale expression (10 milliliter culture volume and more) the constructs were transiently expressed in mammalian cell cells as described in Tom et al., 2007. Briefly, the expression plasmid transfected into HEK293-6E cells and incubated in Fernbach-Flasks or Wave-Bags. Expression was at 37°C for 5 to 6 days in F17 Medium (Invitrogen). 5 g/l Tryptone TN1 (Organotechnie), 1% Ultra-Low IgG FCS (Invitrogen) and 0.5 mM Valproic acid (Sigma) were supplemented after transfection.

### Quantification of expressed Relaxin variants:

For quantification of secreted and purified recombinant Relaxin variants, the commercially available quantification ELISA (R&D Systems, Human Relaxin-2 Quantikine ELISA Kit, catalogue number DRL200) was used according to the manufactures' instructions.

### Activity testing:

CHO K1 cells (ATCC, catalogue number CCL-61) were stably transfected with the cyclic AMP responsive element (CRE) Luciferase reporter gene construct (Biomyx Technology, pHTS-CRE, catalogue number P2100) resulting in a CHO-CRELuciferase cell line.

This cell line was subsequently stably transfected with the human LGR7/RXFP1 receptor (accession numbers NM_021634.2), cloned as 2271 base pair long DNA fragment into the mammalian expression vector pcDNA3.1(-) (Invitrogen, catalogue number V79520), resulting in a CHO-CRE-LGR7 cell line. This cell line was cultivated in D-Mem F12 (Gibco, #31330) 2 mM Glutamax (Gibco, #35050), 100 nM Pyruvat (Gibco, # 11360-070), 20 mM Hepes (Gibco, # 15630), 1% Penicillin-Streptomycin (Gibco, #15140) and 10% fetal calf serum (FCS, Gibco, #11058).

For stimulation, medium was exchanged by OptiMem (Gibco, #11058) + 1% FCS containing different concentrations of the purfified or non-purified recombinant expressed Fc-single chain Relaxin variant proteins (usually starting at a concentration of 100 nM, followed by 1:2 dilutions). As positive control, commercially available recombinant expressed human Relaxin 2 (Genbank Accession number NP_604390.1) was used (R&D Systems, catalogue number 6586-RN-025). Subsequently, cells were incubated for 6 hours in a humified incubator at 5% carbon dioxide at 37°C. After 6 hours cells were tested fo r Luciferase activity using a Luciferase Assay System (Promega, # E1500) and using the Tecan Infinite 500 reader, luminescence mode, 1000 milliseconde integration time, measurement time 30 seconds.

Relative luminescence units were used to determine EC50 values of the different molecules by using the computer program Graph Pad Prism Version 5.

### Immunogenicity testing

Immunogenicity testing is performed by using the computer program NetMHCIIpan (Center for Biological Sequence Analysis; Department of Systems Biology; Technical University of Denmark) which calculates the potential binding affinity of proteins or peptides to MHCII complex. The higher the calculated binding affinity the higher is the risk to induce antibodies directed against the protein or polypeptide of interest.

In vitro determination of mapping T cell epitopes is performed according to the protocol published by Reijonen and Kwok (Reijonen H., Kwok WW. (2003) Use of HLA class II tetramers in tracking antigen-specific T cells and mapping T-cell epitopes. Methods 29:282-288).

### Example 1: IgG2Fc-scRelaxin

lgG2Fc-scRelaxin consists of the single chain variant of human Relaxin 2, in which the C-terminal end of the A-chain and the N-terminal end of the B-chain are connected via a GGGSGGGSG linker. The N-terminal end of the A-chain is connected via a polypeptide as stretcher consisting of the amino acid composition GGSGGSP to the Fc moiety of the human lgG2 molecule. This results in a polypeptide as depicted in SEQ ID NO: 1.

### Example 2: IgG4Fc-scRelaxin

IgG2Fc-scRelaxin consists of the single chain variant of human Relaxin 2, in which the C-terminal end of the A-chain and the N-terminal end of the B-chain are connected via a GGGSGGGSG linker. The N-terminal end of the A-chain is connected via a polypeptide as stretcher consisting of the amino acid composition GGSGGSP to the Fc moiety of the human IgG4 molecule. This results in a polypeptide as depicted in SEQ ID NO: 2.

Further citations:
Hsu, S. Y. (2003). New insights into the evolution of the relaxin-LGR signaling system. Trends Endocrinol Metab 14:303-309
Wilkinson, T. N., Speed, T. P., Tregear, G. W., Bathgate, R. A. (2005).Evolution of the relaxin-like peptide family. BMC Evol Biol 5:14
Hudson P, Haley J, John M, Cronk M, Crawford R, Haralambidis J, Tregear G, Shine J, Niall H. (1983) Structure of a genomic clone encoding biologically active human relaxin. Nature 301: 628-631
Toth, M., Taskinen, P., & Ruskoaho, H. (1996). Relaxin stimulates atrial natriuretic peptide secretion in perfused rat heart. J Endocrinol 150: 487-495
Piedras-Renteria, E. S., Sherwood, O. D., and Best, P. M. (1997). Effects of relaxin on rat atrial myocytes: I. Inhibition of I(to) via PKA-dependent phosphorylation. Am J Physiol 272:H1791-H1797
Bartsch, O., Bartlick, B., and Ivell, R. (2001). Relaxin signaling links tyrosine phosphorylation to phosphodiesterase and adenylyl cyclase activity. Mol Hum Reprod 7:799-809
Bartsch, O., Bartlick, B., and Ivell, R. (2004). Phosphodiesterase 4 inhibition synergizes with relaxin signaling to promote decidualization of human endometrial stromal cells. J Clin Endocrinol Metab 89:324-334
Bani-Sacchi, T., Bigazzi, M., Bani, D., Mannaioni, P. F., and Masini, E. (1995) Relaxin-induced increased coronary flow through stimulation of nitric oxide production. Br J Pharmacol 116:1589-1594
Dschietzig T, Bartsch C, Baumann G, Stangl K. (2006) Relaxin - a pleiotropic hormone and its emerging role for experimental and clinical therapeutics. Pharmacol. Ther. 112:38-56
McGuane JT, Parry LJ. (2005) Relaxin and the extracellularmatrix: Molecular mechanisms of action and implications for cardiovascular disease. Expert. Rev. Mol.Med. 7:1-18
Nistri, S., Chiappini, L., Sassoli, C. and Bani, D. (2003) Relaxin inhibits lipopolysaccharide-induced adhesion of neutrophils to coronary endothelial cells by a nitric oxide-mediated mechanism. FASEB J. 17:2109-2111
Perna AM, Masini E, Nistri S, Briganti V, Chiappini L, Stefano P, Bigazzi M, Pieroni C, Bani Sacchi T, Bani D. (2005) Novel drug development opportunity for relaxin in acute myocardial infarction: evidences from a swine model. FASEB J. 19:1525-1527 Bani, D., Masini, E., Bello, M. G., Bigazzi, M. and Sacchi, T. B. (1998) Relaxin protects against myocardial injury caused by ischemia and reperfusion in rat heart. Am. J. Pathol. 152:1367-1376;
Zhang J, Qi YF, Geng B, Pan CS, Zhao J, Chen L, Yang J, Chang JK, Tang CS. (2005) Effect of relaxin on myocardial ischemia injury induced by isoproterenol. Peptides 26:1632-1639
Teerlink JR, Metra M, Felker GM, Ponikowski P, Voors AA, Weatherley BD, Marmor A, Katz A, Grzybowski J, Unemori E, Teichman SL, Cotter G. (2009) Relaxin for the treatment of patients with acute heart failure (Pre-RELAX-AHF): a multicentre, randomised, placebo-controlled, parallel-group, dose-fi nding phase IIb study. Lancet. 373:1429-1439
Metra M, Teerlink JR, Felker GM, Greenberg BH, Filippatos G, Ponikowski P, Teichman SL, Unemori E, Voors AA, Weatherley BD, Cotter G. (2010) Dyspnoea and worsening heart failure in patients with acute heart failure: results from the Pre-RELAX-AHF study. Eur J Heart Fail. 12:1130-1139
Cosen-Binker LI, Binker MG, Cosen R, Negri G, Tiscornia O. (2006) Relaxin prevents the development of severe acute pancreatitis. World J. Gastroenterol. 12:1558-1568 Santora K, Rasa C, Visco D, Steinetz BG, Bagnell CA. (2007) Antiarthritic effects of relaxin, in combination with estrogen, in rat adjuvant induced arthritis. J. Pharmacol. Exp. Ther. 322:887-893
Bennett RG. (2009) Relaxin and its role in the development and treatment of fibrosis. Transl Res. 154:1-6
Barlos KK, Gatos D, Vasileiou Z, Barlos K. (2010) An optimized chemical synthesis of human relaxin-2. J Pept Sci. 16:200-211.
Park JI, Semyonov J, Yi W, Chang CL, Hsu SY (2008) Regulation of receptor signaling by relaxin A chain motifs: derivation of pan-specific and LGR7-specific human relaxin analogs. J Biol Chem. 283:32099-32109
Shaw JA, Delday MI, Hart AW, Docherty HM, Maltin CA, Docherty K (2002) Secretion of bioactive human insulin following plasmid-mediated gene transfer to nonneuroendocrine cell lines, primary cultures and rat skeletal muscle in vivo. J Endocrinol 172:653-672
Dschietzig T, Teichmann S, Unemori E, Wood S, Boehmer J, Richter C, Baumann G, Stangl K (2009) Intravenous Recombinant Human Relaxin in Compensated Heart Failure: A Safety, Tolerability, and Pharmacodynamic Trial. J Cardiac Fail 5:182-190
WO 97/26265
WO 99/03861
WO 00/06568
WO 00/06569
WO 02/42301
WO 03/095451
WO 01/19355
WO 01/19776
WO 01/19778
WO 01/19780
WO 02/070462
WO 02/070510

## Claims

1. A fusion polypeptide having Relaxin activity comprising in order from the amino-terminus to the carboxy-terminus:
a. an IgG4 Fc domain
b. a stretcher unit, and
c. the scRelaxin polypeptide having the amino acid sequence:

2. A fusion polypeptide having Relaxin activity comprising SEQ ID NO:1 or SEQ ID NO:2.

3. A homodimer of a fusion polypeptide according to claims 1 or 2.

4. A polynucleotide encoding a fusion polypeptide according to claims 1 or 2.

5. A vector comprising a polynucleotide according to claim 4.

6. A host cell comprising a vector according to claim 5 or a polynucleotide according to claim 4.

7. A method of producing a polypeptide according to anyone of claims 1 - 2 or a homodimer according to claim 3 comprising the steps of cultivating a host cell of claim 6 and isolating the polypeptide or homodimer.

8. A pharmaceutical composition comprising a fusion polypeptide according to anyone of claims 1 - 2 or a homodimer according to claim 3.

9. A pharmaceutical composition according to claim 8 or a fusion polypeptide according to anyone of claims 1 - 2 or a homodimer according to claim 3 for use as medicament.

10. A pharmaceutical composition according to claim 8 or 9, a fusion polypeptide according to anyone of claims 1 - 2, or a homodimer according to claim 3 for use as medicament for the treatment of cardiovascular disease, lung disease, fibrotic disorder or kidney disease.

11. The pharmaceutical composition, the fusion polypeptide, or the homodimer for use as medicament for the treatment of a cardiovascular disease according to claim 10 , wherein the cardiovascular disease is selected from the group of diseases comprising coronary heart disease, acute coronary syndrome, heart failure, and myocardial infarction.

12. The pharmaceutical composition, the fusion polypeptide, or the homodimer for use as medicament for the treatment of a heart failure disease according to claim 11 , wherein the heart failure disease is selected from the group of diseases comprising heart failure with reduced ejection fraction (HFrEF), heart failure with preserved ejection fraction (HFpEF), acute decompensated heart failure, right heart failure, left heart failure, global failure, ischemic cardiomyopathy (ICM), dilated cardiomyopathy (DCM), hypertrophic cardiomyopathy (HCM), and congenital heart defects.

## Patentansprüche

1. Fusionspolypeptid mit Relaxin-Aktivität, umfassend nacheinander vom Aminoterminus zum Carboxy-terminus:
a. eine IgG4-Fc-Domäne
b. eine Stretcher-Einheit und
c. das scRelaxin-Polypeptid mit der Aminosäuresequenz:

2. Fusionspolypeptid mit Relaxin-Aktivität, umfassend SEQ ID NO:1 oder SEQ ID NO:2.

3. Homodimer eines Fusionspolypeptids nach Anspruch 1 oder 2.

4. Polynukleotid, codierend ein Fusionspolypeptid nach Anspruch 1 oder 2.

5. Vektor, umfassend ein Polynukleotid nach Anspruch 4.

6. Wirtszelle, umfassend einen Vektor nach Anspruch 5 oder ein Polynukleotid nach Anspruch 4.

7. Verfahren zur Herstellung eines Polypeptids nach einem der Ansprüche 1 - 2 oder eines Homodimers nach Anspruch 3, umfassend die Schritte des Kultivierens einer Wirtszelle nach Anspruch 6 und des Isolierens des Polypeptids bzw. Homodimers.

8. Pharmazeutische Zusammensetzung, umfassend ein Fusionspolypeptid nach einem der Ansprüche 1 - 2 oder ein Homodimer nach Anspruch 3.

9. Pharmazeutische Zusammensetzung nach Anspruch 8 oder Fusionspolypeptid nach einem der Ansprüche 1 - 2 oder Homodimer nach Anspruch 3 zur Verwendung als Arzneimittel.

10. Pharmazeutische Zusammensetzung nach Anspruch 8 oder 9, Fusionspolypeptid nach einem der Ansprüche 1 - 2 oder Homodimer nach Anspruch 3 zur Verwendung als Arzneimittel für die Behandlung einer Herz-Kreislauf-Krankheit, Lungenkrankheit, fibrotischen Erkrankung oder Nierenkrankheit.

11. Pharmazeutische Zusammensetzung, Fusionspolypeptid oder Homodimer zur Verwendung als Arzneimittel für die Behandlung einer Herz-Kreislauf-Krankheit nach Anspruch 10, wobei die Herz-Kreislauf-Krankheit aus der koronare Herzkrankheit, akutes Koronarsyndrom, Herzinsuffizienz und Myokardinfarkt umfassenden Gruppe von Krankheiten ausgewählt ist.

12. Pharmazeutische Zusammensetzung, Fusionspolypeptid oder Homodimer zur Verwendung als Arzneimittel für die Behandlung einer Herzinsuffizienz-Krankheit nach Anspruch 11, wobei die Herzinsuffizienz-Krankheit aus der Herzinsuffizienz mit reduzierter Ejektionsfraktion (Heart Failure with reduced Ejection Fraction, HFrEF), Herzinsuffizienz mit erhaltener Ejektionsfraktion (Heart Failure with preserved Ejection Fraction, HFpEF), akute dekompensierte Herzinsuffizienz, Rechtsherzinsuffizienz, Linksherzinsuffizienz, Globalinsuffizienz, ischämische Kardiomyopathie (ICM), dilatative Kardiomyopathie (DCM), hypertrophe Kardiomyopathie (HCM) und angeborene Herzfehler umfassenden Gruppe von Krankheiten ausgewählt ist.

## Revendications

1. Polypeptide de fusion présentant une activité de Relaxine comprenant dans l'ordre de l'extrémité amino à l'extrémité carboxy :
a. un domaine IgG4 Fc
b. une unité d'extension, et
c. le polypeptide scRelaxine comprenant la séquence d'acides aminées :

2. Polypeptide de fusion présentant une activité de Relaxine comprenant SEQ ID NO:1 ou SEQ ID NO:2.

3. Homodimère d'un polypeptide de fusion selon les revendications 1 ou 2.

4. Polynucléotide codant un polypeptide de fusion selon les revendications 1 ou 2.

5. Vecteur comprenant un polynucléotide selon la revendication 4.

6. Cellule hôte comprenant un vecteur selon la revendication 5 ou polynucléotide selon la revendication 4.

7. Procédé de production d'un polypeptide selon l'une quelconque des revendications 1 à 2 ou d'un homodimère selon la revendication 3 comprenant les étapes de culture d'une cellule hôte selon la revendication 6 et d'isolement du polypeptide ou de l'homodimère.

8. Composition pharmaceutique comprenant un polypeptide de fusion selon l'une quelconque des revendications 1 à 2 ou un homodimère selon la revendication 3.

9. Composition pharmaceutique selon la revendication 8, polypeptide de fusion selon l'une quelconque des revendications 1 à 2 ou homodimère selon la revendication 3 pour utilisation que médicament.

10. Composition pharmaceutique selon la revendication 8 ou 9, polypeptide de fusion selon l'une quelconque des revendications 1 à 2, ou homodimère selon la revendication 3 pour utilisation comme médicament dans le traitement d'une maladie cardio-vasculaire, d'une maladie pulmonaire, d'un trouble fibrotiques ou d'une maladie rénale.

11. Composition pharmaceutique, polypeptide de fusion ou homodimère pour utilisation en tant que médicament dans le traitement d'une maladie cardio-vasculaire selon la revendication 10, où la maladie cardio-vasculaire est choisie dans le groupe de maladies comprenant une maladie coronarienne, un syndrome coronarien aigu, une insuffisance cardiaque et un infarctus du myocarde.

12. Composition pharmaceutique, polypeptide de fusion, ou homodimère pour utilisation en tant que médicament dans le traitement d'une maladie d'insuffisance cardiaque selon la revendication 11, où la maladie d'insuffisance cardiaque est choisie dans le groupe de maladies comprenant les suivantes : insuffisance cardiaque à fraction d'éjection réduite (HFrEF), insuffisance cardiaque à fraction d'éjection préservée (HFpEF), insuffisance ventriculaire droite, insuffisance ventriculaire gauche, insuffisance globale, myocardiopathie ischémique (MCI), myocardiopathie dilatée (MCD), myocardiopathie hypertrophique (MCH) et défauts cardiaques congénitaux.


### SEQUENCE LISTING

<110> Bayer Pharma AG
<120> Fusion polypeptides and uses thereof
<130> BHC 12 1 036
<160> 4
<170> PatentIn version 3.5
<210> 1
   <211> 291
   <212> PRT
   <213> Artificial Sequence
<220>
   <223> human IgG2 scRelaxin - fusionpolypeptide
<400> 1
   Val Glu Cys Pro Pro Cys Pro Ala Pro Pro Val Ala Gly Pro Ser Val
   1               5                   10                  15
   Phe Leu Phe Pro Pro Lys Pro Lys Asp Thr Leu Met Ile Ser Arg Thr
               20                  25                  30
   Pro Glu Val Thr Cys Val Val Val Asp Val Ser His Glu Asp Pro Glu
           35                  40                  45
   Val Gln Phe Asn Trp Tyr Val Asp Gly Met Glu Val His Asn Ala Lys
       50                  55                  60
   Thr Lys Pro Arg Glu Glu Gln Phe Asn Ser Thr Phe Arg Val Val Ser
   65                  70                  75                  80
   Val Leu Thr Val Val His Gln Asp Trp Leu Asn Gly Lys Glu Tyr Lys
                   85                  90                  95
   Cys Lys Val Ser Asn Lys Gly Leu Pro Ala Pro Ile Glu Lys Thr Ile
               100                 105                 110
   Ser Lys Thr Lys Gly Gln Pro Arg Glu Pro Gln Val Tyr Thr Leu Pro
           115                 120                 125
   Pro Ser Arg Glu Glu Met Thr Lys Asn Gln Val Ser Leu Thr Cys Leu
       130                 135                 140
   Val Lys Gly Phe Tyr Pro Ser Asp Ile Ala Val Glu Trp Glu Ser Asn
   145                 150                 155                 160
   Gly Gln Pro Glu Asn Asn Tyr Lys Thr Thr Pro Pro Met Leu Asp Ser
   165 170 175
   Asp Gly Ser Phe Phe Leu Tyr Ser Lys Leu Thr Val Asp Lys Ser Arg
   180 185 190
   Trp Gln Gln Gly Asn Val Phe Ser Cys Ser Val Met His Glu Ala Leu
   195 200 205
   His Asn His Tyr Thr Gln Lys Ser Leu Ser Leu Ser Pro Gly Lys Gly
   210 215 220
   Gly Ser Gly Gly Ser Pro Gln Leu Tyr Ser Ala Leu Ala Asn Lys Cys
   225 230 235 240
   Cys His Val Gly Cys Thr Lys Arg Ser Leu Ala Arg Phe Cys Gly Gly
   245 250 255
   Gly Ser Gly Gly Gly Ser Gly Ser Trp Met Glu Glu Val Ile Lys Leu
   260 265 270
   Cys Gly Arg Glu Leu Val Arg Ala Gln Ile Ala Ile Cys Gly Met Ser
   275 280 285
   Thr Trp Ser
   290
<210> 2
   <211> 292
   <212> PRT
   <213> Artificial Sequence
<220>
   <223> human IgG4 scRelaxin fusion polypeptide
<400> 2
   Pro Pro Cys Pro Ser Cys Pro Ala Pro Glu Phe Leu Gly Gly Pro Ser
   1               5                   10                  15
   Val Phe Leu Phe Pro Pro Lys Pro Lys Asp Thr Leu Met Ile Ser Arg
               20                  25                  30
   Thr Pro Glu Val Thr Cys Val Val Val Asp Val Ser Gln Glu Asp Pro
           35                  40                  45
   Glu Val Gln Phe Asn Trp Tyr Val Asp Gly Val Glu Val His Asn Ala
       50                  55                  60
   Lys Thr Lys Pro Arg Glu Glu Gln Phe Asn Ser Thr Tyr Arg Val Val
   65                  70                  75                  80
   Ser Val Leu Thr Val Leu His Gln Asp Trp Leu Asn Gly Lys Glu Tyr
                   85                  90                  95
   Lys Cys Lys Val Ser Asn Lys Gly Leu Pro Ser Ser Ile Glu Lys Thr
               100                 105                 110
   Ile Ser Lys Ala Lys Gly Gln Pro Arg Glu Pro Gln Val Tyr Thr Leu
           115                 120                 125
   Pro Pro Ser Gln Glu Glu Met Thr Lys Asn Gln Val Ser Leu Thr Cys
       130                 135                 140
   Leu Val Lys Gly Phe Tyr Pro Ser Asp Ile Ala Val Glu Trp Glu Ser
   145                 150                 155                 160
   Asn Gly Gln Pro Glu Asn Asn Tyr Lys Thr Thr Pro Pro Val Leu Asp
                   165                 170                 175
   Ser Asp Gly Ser Phe Phe Leu Tyr Ser Arg Leu Thr Val Asp Lys Ser
               180                 185                 190
   Arg Trp Gln Glu Gly Asn Val Phe Ser Cys Ser Val Met His Glu Ala
           195                 200                 205
   Leu His Asn His Tyr Thr Gln Lys Ser Leu Ser Leu Ser Leu Gly Lys
       210                 215                 220
   Gly Gly Ser Gly Gly Ser Pro Gln Leu Tyr Ser Ala Leu Ala Asn Lys
   225                 230                 235                 240
   Cys Cys His Val Gly Cys Thr Lys Arg Ser Leu Ala Arg Phe Cys Gly
                   245                 250                 255
   Gly Gly Ser Gly Gly Gly Ser Gly Ser Trp Met Glu Glu Val Ile Lys
               260                 265                 270
   Leu Cys Gly Arg Glu Leu Val Arg Ala Gln Ile Ala Ile Cys Gly Met
           275                 280                 285
   Ser Thr Trp Ser
       290
<210> 3
   <211> 873
   <212> DNA
   <213> Artificial Sequence
<220>
   <223> polynucleotid efor human IgG2 scRelaxin fusion polypeptide
<400> 3
   gtggaatgcc cgccgtgccc ggcgccgccg gtggcgggcc cgagcgtgtt tctgtttccg      60
   ccgaaaccga aagataccct gatgattagc cgcaccccgg aagtgacctg cgtggtggtg     120
   gatgtgagcc atgaagatcc ggaagtgcag tttaactggt atgtggatgg catggaagtg     180
   cataacgcga aaaccaaacc gcgcgaagaa cagtttaaca gcacctttcg cgtggtgagc     240
   gtgctgaccg tggtgcatca ggattggctg aacggcaaag aatataaatg caaagtgagc     300
   aacaaaggcc tgccggcgcc gattgaaaaa accattagca aaaccaaagg ccagccgcgc     360
   gaaccgcagg tgtataccct gccgccgagc cgcgaagaaa tgaccaaaaa ccaggtgagc     420
   ctgacctgcc tggtgaaagg cttttatccg agcgatattg cggtggaatg ggaaagcaac     480
   ggccagccgg aaaacaacta taaaaccacc ccgccgatgc tggatagcga tggcagcttt     540
   tttctgtata gcaaactgac cgtggataaa agccgctggc agcagggcaa cgtgtttagc     600
   tgcagcgtga tgcatgaagc gctgcataac cattataccc agaaaagcct gagcctgagc     660
   ccgggcaaag gcggcagcgg cggcagcccg cagctgtata gcgcgctggc gaacaaatgc     720
   tgccatgtgg gctgcaccaa acgcagcctg gcgcgctttt gcggcggcgg cagcggcggc     780
   ggcagcggca gctggatgga agaagtgatt aaactgtgcg gccgcgaact ggtgcgcgcg     840
   cagattgcga tttgcggcat gagcacctgg agc                                  873
<210> 4
   <211> 876
   <212> DNA
   <213> Artificial Sequence
<220>
   <223> polynucleotid eencoding human IgG4 scRelaxin fusion polypeptide
<400> 4
   ccgccgtgcc cgagctgccc ggcgccggaa tttctgggcg gcccgagcgt gtttctgttt 60
   ccgccgaaac cgaaagatac cctgatgatt agccgcaccc cggaagtgac ctgcgtggtg 120
   gtggatgtga gccaggaaga tccggaagtg cagtttaact ggtatgtgga tggcgtggaa 180
   gtgcataacg cgaaaaccaa accgcgcgaa gaacagttta acagcaccta tcgcgtggtg 240
   agcgtgctga ccgtgctgca tcaggattgg ctgaacggca aagaatataa atgcaaagtg 300
   agcaacaaag gcctgccgag cagcattgaa aaaaccatta gcaaagcgaa aggccagccg 360
   cgcgaaccgc aggtgtatac cctgccgccg agccaggaag aaatgaccaa aaaccaggtg 420
   agcctgacct gcctggtgaa aggcttttat ccgagcgata ttgcggtgga atgggaaagc 480
   aacggccagc cggaaaacaa ctataaaacc accccgccgg tgctggatag cgatggcagc 540
   ttttttctgt atagccgcct gaccgtggat aaaagccgct ggcaggaagg caacgtgttt 600
   agctgcagcg tgatgcatga agcgctgcat aaccattata cccagaaaag cctgagcctg 660
   agcctgggca aaggcggcag cggcggcagc ccgcagctgt atagcgcgct ggcgaacaaa 720
   tgctgccatg tgggctgcac caaacgcagc ctggcgcgct tttgcggcgg cggcagcggc 780
   ggcggcagcg gcagctggat ggaagaagtg attaaactgt gcggccgcga actggtgcgc 840
   gcgcagattg cgatttgcgg catgagcacc tggagc 876